(19) Europäisches Patentamt
European Patent Office
Office européen des brevets

(11) EP 1 128 804 B1

(12) **EUROPÄISCHE PATENTSCHRIFT**

(45) Veröffentlichungstag und Bekanntmachung des Hinweises auf die Patenterteilung:
**20.10.2004 Patentblatt 2004/43**

(21) Anmeldenummer: **99953976.0**

(22) Anmeldetag: **09.11.1999**

(51) Int Cl.[7]: **A61K 7/42**, A61K 7/13

(86) Internationale Anmeldenummer:
**PCT/EP1999/008568**

(87) Internationale Veröffentlichungsnummer:
**WO 2000/028957 (25.05.2000 Gazette 2000/21)**

(54) **NEUE VERWENDUNG VON UV-FILTERN, VERFAHREN ZUR FÄRBUNG KERATINISCHER FASERN UND MITTEL FÜR DIESE VERFAHREN**

NOVEL USE OF UV FILTERS, METHOD FOR COLOURING KERATINIC FIBRES AGENTS FOR REALISING THIS METHOD

NOUVELLE UTILISATION DE FILTRES U.V., PROCEDE DE COLORATION DE FIBRES KERATINIQUES ET AGENTS UTILISES POUR METTRE LEDIT PROCEDE EN OEUVRE

(84) Benannte Vertragsstaaten:
**AT BE CH CY DE DK ES FI FR GB GR IE IT LI LU MC NL PT SE**

(30) Priorität: **18.11.1998 DE 19853111**

(43) Veröffentlichungstag der Anmeldung:
**05.09.2001 Patentblatt 2001/36**

(73) Patentinhaber: **Henkel Kommanditgesellschaft auf Aktien**
**40589 Düsseldorf-Holthausen (DE)**

(72) Erfinder:
- **EHLERT, Manuela**
**D-51379 Leverkusen (DE)**
- **BERNECKER, Ullrich**
**D-52393 Hürtgenwald (DE)**
- **HÖFFKES, Horst**
**D-40595 Düsseldorf (DE)**
- **HOLLENBERG, Detlef**
**D-40699 Erkrath (DE)**
- **MEINIGKE, Bernd**
**D-51381 Leverkusen (DE)**

(56) Entgegenhaltungen:
**EP-A- 0 437 006**
**WO-A-95/05797**
**WO-A-96/03968**
**DE-A- 2 046 818**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 128 804 B1

Printed by Jouve, 75001 PARIS (FR)

## Beschreibung

Die Erfindung betrifft die Verwendung von UV-Filtern zur Verbesserung der Waschechtheit von Färbungen keratinischer Fasern, Verfahren zur Färbung keratinischer Fasern sowie Mittel zur Anwendung in diesen Verfahren.

Im Rahmen der Produkte, die zur kosmetischen Behandlung des menschlichen Körpers bereitgestellt werden, spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen drei Typen von Mitteln zur Veränderung der Haarfarbe von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoff-vorprodukten eingesetzt werden; in vielen Fällen werden weiterhin direktziehende Farbstoffe zur Nuancierung verwendet. Weisen die im Verlauf der Farbausbildung gebildeten bzw. direkt eingesetzten Farbstoffe deutlich unterschiedliche Echtheiten (z. B. UV-Stabilität, Schweißechtheit, Waschechtheit etc.) auf, so kann es mit der Zeit zu einer erkennbaren und daher unerwünschten Farbverschiebung kommen. Dieses Phänomen tritt verstärkt auf, wenn die Frisur Haare oder Haarzonen unterschiedlichen Schädigungsgrades aufweist. Ein Beispiel dafür sind lange Haare, bei denen die lange Zeit allen möglichen Umwelteinflüssen ausgesetzten Haarspitzen in der Regel deutlich stärker geschädigt sind als die relativ frisch nachgewachsenen Haarzonen.

Für temporäre Färbungen werden üblicherweise Färbe- oder Tönungsmittel verwendet. die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden. Auch hier können dann Probleme hinsichtlich der Echtheit der Färbungen auftreten.

Es hat daher nicht an Anstrengungen gefehlt, die Echtheit von Färbungen keratinischer Fasern zu verbessern. Eine Entwicklungsrichtung ist die Optimierung der Farbstoffe selbst bzw. die Synthese neuer, modifizierter Farbstoffmoleküle. Eine weitere Entwicklungsrichtung ist die Suche nach Zusätzen für die Färbemittel, um die Echtheit der Färbungen zu erhöhen. Eine bekannte Problemlösung ist, dem Färbemittel UV-Filter zuzusetzen. Diese Filtersubstanzen werden beim Färbeprozeß zusammen mit dem Farbstoff auf das Haar aufgebracht, wodurch in vielen Fällen eine deutliche Steigerung der Stabilität der Färbung gegen die Einwirkung von Tages- oder Kunstlicht erzielt wird.

Es wurde nun überraschenderweise gefunden, daß durch den Einsatz von UV-Filtern auch die Waschechtheit von Färbungen keratinischer Fasern signifikant gesteigert werden kann. Unter Waschechtheit im Sinne der Erfindung ist die Erhaltung der ursprünglichen Färbung hinsichtlich Nuance und/oder Intensität zu verstehen, wenn die keratinische Faser dem wiederholten Einfluß von wäßrigen Mitteln, insbesondere tensidhaltigen Mitteln wie Shampoos, ausgesetzt wird.

Ein erster Gegenstand der vorliegenden Erfindung ist daher die Verwendung von UV-Filtem zur Verbesserung der Waschechtheit von Färbungen keratinischer Fasern.

Unter keratinischen Fasem werden erfindungsgemäß Pelze, Wolle, Federn und insbesondere menschliche Haare verstanden.

Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300

nm, sind besonders bevorzugt.
Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestem, Benzimidazolen und o-Aminobenzoesäureestern.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul®M 40, Uvasorb®MET, Neo Heliopan®BB, Eusolex®4360), 2-Phenylbenzimidazol-5-sulfonsäure und deren Kalium-, Natrium- und Triethanolaminsalze (Phenylbenzimidazole sulfonic acid; Parsol®HS; Neo Heliopan®Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol®1789, Eusolex®9020), α-(2-Oxoborn-3-yliden)-toluol-4-sulfonsäure und deren Salze, ethoxylierte 4-Aminobenzoesäure-ethylester (PEG-25 PABA; Uvinul®P 25), 4-Dimethylaminobenzoesäure-2-ethylhexylester (Octyl Dimethyl PABA; Uvasorb®DMO. Escalol®507, Eusolex®6007), Salicylsäure-2-ethylhexylester (Octyl Salicylat; Escalol®587, Neo Heliopan®OS, Uvinul®O18), 4-Methoxyzimtsäure-isopentylester (Isoamyl p-Methoxycinnamate; Neo Heliopan®E 1000), 4-Methoxyzimtsäure-2-ethylhexyl-ester (Octyl Methoxycinnamate; Parsol®MCX, Escalol®557, Neo Heliopan®AV), 2-Hydroxy-4-methoxybenzophenon-5-sulfonsäure und deren Natriumsalz (Benzophenone-4; Uvinul®MS 40; Uvasorb®S 5), 3-(4'-Methylbenzyliden)-D,L-Campher (4-Methylbenzylidene camphor; Parsol®5000, Eusolex®6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb®20 H, Uvinul®400), 1,1'-Diphenylacrylonitrilsäure-2-ethylhexyl-ester (Octocrylene; Eusolex®OCR, Neo Heliopan®Type 303, Uvinul®N 539 SG), o-Aminobenzoesäure-menthylester (Menthyl Anthranilate; Neo Heliopan®MA), 2,2',4,4'-Tetrahydroxybenzophenon (Benzophenone-2; Uvinul®D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2'-cthylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D, L-Campher.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, daß bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Diese UV-Filter weisen die allgemeine Struktur U - Q auf.

Der Strukturteil U steht dabei für eine UV-Strahlen absorbierende Gruppe. Diese Gruppe kann sich im Prinzip von den bekannten, im Kosmetikbereich einsetzbaren. oben genannten UV-Filtern ableiten, in dem eine Gruppe, in der Regel ein Wasserstoffatom. des UV-Filters durch eine kationische Gruppe Q, insbesondere mit einer quartären Aminofunktion, ersetzt wird.
Verbindungen, von denen sich der Strukturteil U ableiten kann, sind beispielsweise

- substituierte Benzophenone,
- p-Aminobenzoesäureester,
- Diphenylacrylsäureester,

- Zimtsäureester,
- Salicylsäureester,
- Benzimidazole und
- o-Aminobenzoesäureester.

Strukturteile U, die sich vom Zimtsäureamid oder vom N,N-Dimethylamino-benzoesäureamid ableiten, sind erfindungsgemäß bevorzugt.

Die Strukturteile U können prinzipiell so gewählt werden, daß das Absorptionsmaximum der UV-Filter sowohl im UVA(315-400 nm)-, als auch im UVB(280-315nm)- oder im UVC(<280 nm)-Bereich liegen kann. UV-Filter mit einem Absorptionsmaximum im UVB-Bereich, insbesondere im Bereich von etwa 280 bis etwa 300 nm, sind besonders bevorzugt.

Weiterhin wird der Strukturteil U, auch in Abhängigkeit von Strukturteil Q, bevorzugt so gewählt, daß der molare Extinktionskoeffizient des UV-Filters am Absorptionsmaximum oberhalb von 15 000, insbesondere oberhalb von 20000, liegt.

Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so daß der Strukturteil U einen der vier Substituenten des positiv geladenen Stickstoffatomes darstellt. Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.

Vorteilhafterweise hat die Gruppe Q die allgemeine Struktur $-(CH_2)_X-N^+R^1R^2R^3\ X^-$, in der x steht für eine ganze Zahl von 1 bis 4, $R^1$ und $R^2$ unabhängig voneinander stehen für $C_{1-4}$-Alkylgruppen, $R^3$ steht für eine $C_{1-22}$-Alkylgruppe oder eine Benzylgruppe und $X^-$ für ein physiologisch verträgliches Anion. Im Rahmen dieser allgemeinen Struktur steht x bevorzugt für die die Zahl 3, $R^1$ und $R^2$ jeweils für eine Methylgruppe und $R^3$ entweder für eine Methylgruppe oder eine gesättigte oder ungesättigte, lineare oder verzweigte Kohlenwasserstoffkette mit 8 bis 22, insbesondere 10 bis 18, Kohlenstoffatomen.

Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat.

Zwei bevorzugte UV-Filter mit kationischen Gruppen sind die als Handelsprodukte erhältlichen Verbindungen Zimtsäureamidopropyl-trimethylammoniumchlorid (Incroquat®UV-283) und Dodecyl-dimethylaminobenzamidopropyl-dimethylammoniumtosylat (Escalol® HP 610).

Selbstverständlich umfaßt die erfindungsgemäße Lehre auch die Verwendung einer Kombination von mehreren UV-Filtern. Im Rahmen dieser Ausführnrgsform ist die Kombination mindestens eines wasserunlöslichen UV-Filters mit mindestens einem UV-Filter mit einer kationischen Gruppe bevorzugt.

Die UV-Filter sind in den erfindungsgemäß verwendeten Mitteln üblicherweise in Mengen 0,1-5 Gew.-%, bezogen auf das gesamte Mittel, enthalten. Mengen von 0,4-2,5 Gew.-% sind bevorzugt.

Die erfindungsgemäße Wirkung der UV-Filter läßt sich weiter steigern. wenn diese in Kombination mit mindestens einem Mono-, Di- oder Oligosaccharid eingesetzt werden. Bevorzugte Saccharide sind Glucose, Galactose, Fructose, Mannose, Fruchtzucker und Lactose. Glucose ist besonders bevorzugt.

Die Saccharide sind in den erfindungsgemäßen Mitteln in Mengen von 0,1-10, insbesondere 1-3 Gew.-%, bezogen auf die jeweilige Formulierung, enthalten

Ebenfalls signifikant steigern läßt sich die erfindungsgemäße Wirkung der UV-Filter. wenn diese in Kombination mit einem Spreitmittel eingesetzt werden.

Spreitmittel im Sinne der Erfindung sind Substanzen, die eine gleichmäßige Verteilung von Wirkstoffen, insbesondere von UV-Filtern, auf der Oberfläche der keratinischen Faser bewirken.

Bevorzugte Spreitmittel sind Ölkomponenten.

Erfindungsgemäß geeignete Ölkomponenten sind prinzipiell alle wasserunlöslichen Öle und Fettstoffe sowie deren Mischungen mit festen Paraffinen und Wachsen. Als wasserunlöslich werden erfindungsgemäß solche Stoffe definiert, deren Löslichkeit in Wasser bei 20 °C kleiner als 0,1 Gew.-% beträgt. Der Schmelzpunkt der einzelnen Öloder Fettkomponenten liegt bevorzugt unterhalb von etwa 40 °C. Öl- und Fettkomponenten, die bei Raumtemperatur, d. h. unterhalb von 25 °C flüssig sind, können erfindungsgemäß besonders bevorzugt sein. Bei Verwendung mehrerer Öl- und Fettkomponenten sowie ggf. festen Paraffinen und Wachsen ist es in der Regel jedoch auch ausreichend, wenn die Mischung der Öl- und Fettkomponenten sowie ggf. Paraffine und Wachse diesen Bedingungen genügt.

Eine bevorzugte Gruppe von Ölkomponenten sind pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls.

Geeignet sind aber auch andere Triglyceridöle wie die flüssigen Anteile des Rindertalgs sowie synthetische Triglyceridöle.

Eine weitere, besonders bevorzugte Gruppe erfindungsgemäß als Spreitmittel einsetzbarer Verbindungen sind flüssige Paraffinöle und synthetische Kohlenwasserstoffe sowie Di-nalkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-nundecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-noctylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.

Ebenfalls erfindungsgemäß einsetzbare Ölkomponenten sind Fettsäure- und Fettalkoholester. Bevorzugt sind die Monoester der Fettsäuren mit Alkoholen mit 3 bis 24 C-Atomen. Bei dieser Stoffgruppe handelt es sich um die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol® SN), Stearinsäure-2-ethylhexylester (Cetiol® 868), Cetyloleat, Glycerintricaprylat, Kokosfettalkohol-caprinat/-caprylat und n-Butylstearat.

Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykoldioleat, Ethylenglykol-di-isotridecanoat, Propylenglykol-di(2-ethylhexanoat), Propylenglykol-di-isostearat, Propylenglykol-di-pelargonat, Butandiol-di-isostearat und Neopentylglykoldi-capylat erfindungsgemäß verwendbare Ölkomponenten dar, ebenso komplexe Ester wie z. B. das Diacetyl- glycerinmonostearat.

Weiterhin können auch Fettalkohole mit 8 bis 22 C-Atomen als erfindungsgemäß wirkende Spreitmittel eingesetzt werden. Die erfindungsgemäß verwendeten Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestem erzeugt werden, und somit ein Gemisch von unterschiedlichen Fettalkoholen darstellen.

Erfindungsgemäß verwendbare Spreitmittel sind schließlich auch Silikonöle, insbesondere Dialkyl- und Alkylarylsiloxane, wie beispielsweise Dimethylpolysiloxan und Methylphenylpolysiloxan, sowie deren alkoxylierte und quatemierte Analoga. Beispiele für solche Silikone sind die von Dow Coming unter den Bezeichnungen DC 190, DC 200 und DC 1401 vertriebenen Produkte sowie die Handelsprodukte DC 344 und DC 345 von Dow Corning, Q2-7224 (Hersteller: Dow Coming; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80).

Die Gesamtmenge an Spreitmitteln in den erfindungsgemäß verwendeten Zubereitungen beträgt üblicherweise 0,5 - 20 Gew.-%, bezogen auf die gesamte Zubereitung. Mengen von 1 - 5 Gew.-% sind erfindungsgemäß bevorzugt.

Der positive Effekt der Spreitmittel wird noch verstärkt, wenn sie zusammen mit kationischen oder anionischen Tensiden eingesetzt werden.

Beispiele für die im Rahmen der vorliegenden Erfindung verwendbaren kationischen Tenside sind insbesondere quartäre Ammoniumverbindungen. Bevorzugt sind Ammoniumhalogenide wie Alkyltrimethylammoniumchloride, Dialkyldi-methylammoniumchloride und Trialkylmethylammoniumchloride, z. B. Cetyltrimethyl-ammoniumchlorid, Stearyltrimethylammoniumchlorid, Distearyldimethylammonium-chlorid, Lauryldimethylammoniumchlorid, Lauryldimethylbenzylammoniumchlorid und Tricetylmethylammoniumchlorid. Alkylamidoamine, insbesondere Fettsäureamidoamine wie das unter der Bezeichnung Tego Amid®S 18 erhältliche Stearylamidopropyldimethylamin, zeichnen sich weiterhin durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex® vertriebenen Methyl-hydroxyalkyl-dialkoyloxyalkyl-ammonium-methosulfate sowie die entsprechenden Produkte, die unter dem Warenzeichen Dehyquart® im Handel erhältlich sind. Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Als anionische Tenside eignen sich alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure. Besonders bevorzugt sind die anionischen Tenside, die mindestens eine Carboxylat-Gruppe enthalten.

Es wurde gefunden, daß die waschechtheitserhöhende Wirkung der UV-Filter sowohl dann auftritt, wenn der UV-Filter zusammen mit dem eigentlichen Färbemittel auf die keratinische Faser aufgebracht wird, als auch dann, wenn der UV-Filter unmittelbar nach dem Färbevorgang mit einer gesonderten Formulierung aufgebracht wird.

Ein zweiter Gegenstand der Erfindung ist daher ein Verfahren zur Färbung keratinischer Fasern in üblicher Weise mit einem Färbemittel, dadurch gekennzeichnet, daß das Färbemittel weiterhin zur Erhöhung der Waschechtheit der Färbung einen UV-Filter enthält. Auch hinsichtlich der erfindungsgemäßen Verfahren stellt das menschliche Haar den bevorzugten Typ der keratinischen Faser dar.

Die Zusammensetzung des Färbemittels unterliegt keinen prinzipiellen Einschränkungen.

Als Farbstoff(vorprodukt)e können

- Oxidationsfarbstoffvorprodukte vom Entwickler- und Kuppler-Typ,
- natürliche und synthetische direktziehende Farbstoffe und
- Vorstufen naturanaloger Farbstoffe, wie Indol- und Indolin-Derivate.

sowie Mischungen von Vertretern einer oder mehrerer dieser Gruppen eingesetzt werden.

Als Oxidationsfarbstoffvorprodukte vom Entwickler-Typ werden üblicherweise primäre aromatische Amine mit einer weiteren, in para- oder ortho-Position befindlichen. freien oder substituierten Hydroxy- oder Aminogruppe, Diaminopyridinderivate. heterocyclische Hydrazone, 4-Aminopyrazolonderivate sowie 2,4,5,6-Tetraaminopyrimidin und dessen Derivate eingesetzt. Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2.5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylaminomethyl-4-aminophenol, 4,4'-Diaminodiphenylamin, 4-Amino-3-fluorphenol, 2-Aminomethyl-4-aminophenol, 2-Hydroxymethyl-4-aminophenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol sowie 4,5-Diaminopyrazol-Derivate nach EP 0 740 741 bzw. WO 94/08970 wie z. B. 4,5-Diamino-1-(2'-hydroxyethyl)-pyrazol. Besonders bevorzugt sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin.

Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivate verwendet. Erfindungsgemäß bevorzugte Kupplerkomponenten sind 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2.6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2.6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 3-Amino-6-methoxy-2-methylaminophenol, 2-Hydroxy-4-aminophenoxyethanol, 2-Methyl-5-(2-hydroxyethylamino)-phenol und 2,6-Dihydroxy-3,4-dimethylpyridin. Besonders bevorzugt sind 1-Naphthol, 1,5-, 2,7- und 1,7-Dihydroxynaphthalin, 5-Amino-2-methylphenol, Resorcin,

4-Chlorresorcin, 2-Chlor-6-methyl-3-aminophenol, 2-Methylresorcin, 5-Methylresorcin, 2,5-Dimethylresorcin, 2,6-Dihydroxy-3,4-diaminopyridin.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Sowohl die Oxidationsfarbstoffvorprodukte als auch die direktziehenden Farbstoffe sind in den erfindungsgemäßen Mitteln bevorzugt in Mengen von 0,01 bis 20 Gew.-%. vorzugsweise 0,5 bis 5 Gew.-%, jeweils bezogen auf das gesamte Mittel, enthalten.

Als Vorläufer natürlicher Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorläufer natürlicher Haarfarbstoff geeignet sind Derivate des 5.6-Dihydroxyindolins der Formel (IIa),

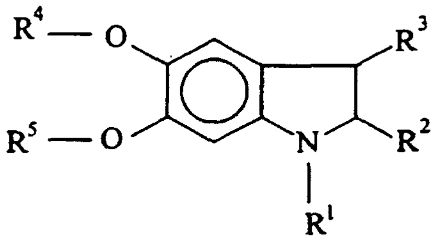

(IIa)

in der unabhängig voneinander

$R^1$ steht für Wasserstoff, eine C1-C4-Alkylgruppe oder eine C1-C4-Hydroxy-alkylgruppe,

$R^2$ steht für Wasserstoff oder eine -COOH-Gruppe, wobei die -COOH-Gruppe auch als Salz mit einem physiologisch verträglichen Kation vorliegen kann,

$R^3$ steht für Wasserstoff oder eine C1-C4-Alkylgruppe,

$R^4$ steht für Wasserstoff, eine C1-C4-Alkylgruppe oder eine Gruppe -CO-$R^6$, in der

$R^6$ steht für eine C1-C4-Alkylgruppe, und

$R^5$ steht für eine der unter $R^4$ genannten Gruppen,

sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin. N-Ethyl-5,6-dihydroxyindolin, N-Propyl-5,6-dihydroxyindolin, N-Butyl-5,6-dihydroxyindolin und insbesondere das 5,6-Dihydroxyindolin.

Besonders gut als Vorläufer natürlicher Haarfarbstoff geeignet sind weiterhin Derivate des 5,6-Dihydroxyindols der Formel (IIb),

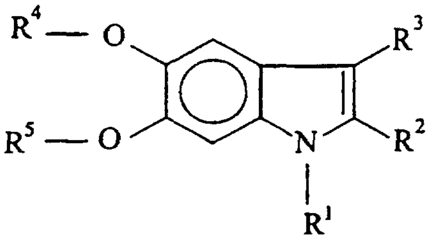

(IIb)

in der unabhängig voneinander

$R^1$ steht für Wasserstoff, eine C1-C4-Alkylgruppe oder eine C1-C4-Hydroxyalkylgruppe,

$R^2$ steht für Wasserstoff oder eine -COOH-Gruppe, wobei die -COOH-Gruppe auch als Salz mit einem physiologisch verträglichen Kation vorliegen kann,

$R^3$ steht für Wasserstoff oder eine C1-C4-Alkylgruppe,

$R^4$ steht für Wasserstoff, eine C1-C4-Alkylgruppe oder eine Gruppe -CO-$R^6$, in der

$R^6$ steht für eine C1-C4-Alkylgruppe, und

$R^5$ steht für eine der unter $R^4$ genannten Gruppen,

sowie physiologisch verträgliche Salze dieser Verbindungen mit einer organischen oder anorganischen Säure. Besonders bevorzugte Derivate des Indols sind 5,6-Dihydroxyindol, N-Methyl-5,6-dihydroxyindol, N-Ethyl-5,6-dihydroxyindöl, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen Färbemitteln üblicherweise in Mengen von 0,05-10 Gew.-%, vorzugsweise 0,2-5 Gew.-% enthalten.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere $\alpha$-Aminocarbonsäuren und $\omega$-Aminocarbonsäuren. Unter den $\alpha$-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt. Die entsprechenden Mittel enthalten die Aminosäure bzw. das Oligopeptid bevorzugt in Mengen von 0,1 bis 10 Gew.-%, insbesondere 1 bis 4 Gew.-%, bezogen auf das gesamte Mittel.

Es ist nicht erforderlich, daß die Oxidationsfarbstoffvorprodukte, die direktziehenden Farbstoffe oder die Vorstufen naturanaloger Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York. Basel. 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e. V., Mannheim, Bezug genommen.

Zur Herstellung der Färbemittel werden die oben genannten zwingenden und fakultativen Bestandteile in einen geeigneten wasserhaltigen Träger eingearbeitet. Sind solche Träger sind z. B. Cremes, Emulsionen, Gele oder auch tensidhaltige schäumende Lösungen, z. B. Shampoos, Schaumaerosole oder andere Zubereitungen, die für die Anwendung auf dem Haar geeignet sind.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Weiterhin können die Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Bezüglich der anionischen und kationischen Tenside wird auf das oben gesagte verwiesen.

Als zwitterionische Tenside werden solche oberflächenaktiven Verbindungen bezeichnet. die im Molekül mindestens eine quartäre Ammoniumgruppe und mindestens eine - $COO^{(-)}$- oder -$SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethyl-ammoniumglycinate, beispielsweise das Kokosacylamino-propyl-dimethylammonium-glycinat, und 2-Alkyl-3-carboxymethyl-3-hydroxyethylimidazoline mit jeweils 8 bis 18 C-Atomen in der Alkyl- oder Acylgruppe sowie das Kokosacyl-aminoethyl-hydroxyethylcarboxymethylglycinat. Ein bevorzugtes zwitter-ionisches Tensid ist das unter der CTFA-Bezeichnung Cocamidopropyl Betaine bekann-te Fettsäureamid-Derivat.

Unter ampholytischen Tensiden werden solche oberflächenaktiven Verbindungen verstanden, die außer einer C8-C18-Alkyl- oder -Acylgruppe im Molekül mindestens eine freie Aminogruppe und mindestens eine -COOH- oder -$SO_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C12-18-Acylsarcosin.

Nichtionische Tenside enthalten als hydrophile Gruppe z. B. eine Polyolgruppe, eine Polyalkylenglykolethergruppe oder eine Kombination aus Polyol- und Polyglykol-ethergruppe. Solche Verbindungen sind beispielsweise:

- Anlagerungsprodukte von 2 bis 30 Mol Ethylenoxid und / oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C12-C22-Fettsäuremono- und -diester von Anlagerungsprodukten von 1 bis 30 Mol Ethylenoxid an Glycerin,
- C8-C22-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise:

- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quatemären Gruppen, Dimethyldiallylammoniumchlorid-Polymere, Acrylamid-Dimethyldiallylammoniumchiorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere,

- anionische Polymere wie beispielsweise Polyacrylsäuren, vernetzte Polyacrylsäuren. Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-. Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes, wie beispielsweise $\alpha$- und $\beta$-Hydroxycarbonsäuren
- Wirkstoffe wie Panthenol, Pantothensäure, Allantoin, Pyrrolidoncarbonsäuren und deren Salze, Pflanzenextrakte und Vitamine,
- Cholesterin,
- Konsistenzgeber wie Zuckerester, Polyolester oder Polyolalkylether,
- Fette und Wachse wie Walrat, Bienenwachs und Montanwachs,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, $N_2O$, Dimethylether, $CO_2$ und Luft,
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.
Bezüglich weiterer fakultativer Komponenten sowie die eingesetzten Mengen dieser Komponenten wird ausdrücklich auf die dem Fachmann bekannten einschlägigen Handbücher, z. B. K. Schrader, Grundlagen und Rezepturen der Kosmetika, 2. Auflage, Hüthig Buch Verlag, Heidelberg, 1989, verwiesen.

Erfolgt die Ausbildung der eigentlichen Haarfarben in Rahmen eines oxidativen Prozesses, so können übliche Oxidationsmittel wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen. Dabei können die Enzyme sowohl zur Erzeugung von oxidierenden Per-Verbindungen eingesetzt werden, als auch zu Verstärkung der Wirkung einer geringen Menge vorhandener Oxidationsmittel. Beispiele für enzymatische Verfahren sind die Verwendung von Laccasen sowie die Verstärkung der Wirkung geringer Mengen (z. B. 1 % und weniger, bezogen auf das gesamte Mittel) Wasserstoffperoxid durch Peroxidasen.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.
Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer

weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt. wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Unabhängig davon, welches der oben genannten Vorgehen im Rahmen des erfindungsgemäßen Verfahrens gewählt wird, kann die Ausbildung der Färbung dadurch unterstützt und gesteigert werden, daß dem Mittel bestimmte Metallionen zugesetzt werden. Solche Metallionen sind beispielsweise $Zn^{2+}$, $Cu^{2+}$, $Fe^{2+}$, $Fe^{3+}$, $Mn^{2+}$, $Mn^{4+}$, $Li^{-}$, $Mg^{2+}$, $Ca^{2-}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Wie bereits oben ausgeführt, kann das Aufbringen des UV-Filters zur Erhöhung der Waschechtheit der Färbungen keratinischen Fasern auch in einem getrennten Schritt im Anschluß an den eigentlichen Färbevorgang erfolgen.

Ein dritter Gegenstand der Erfindung ist somit ein Verfahren zur Färbung keratinischer Fasern, dadurch gekennzeichnet, daß in einem ersten Schritt die keratinische Faser in üblicher Weise gefärbt wird und in einem zweiten Schritt ein Mittel aufgetragen wird, das zur Erhöhung der Waschechtheit der Färbung mindestens einen UV-Filter enthält und gewünschtenfalls nach einer Einwirkzeit von wenigen Sekunden bis etwa 20 Minuten wieder ausgespült wird.

Unter dem Begriff "in üblicher Weise gefärbt" wird im Rahmen der Anmeldung das jedem Fachmann bekannte Vorgehen verstanden, auf das, gegebenenfalls angefeuchtete, Haar ein Färbemittel aufzubringen und dieses für eine Zeit zwischen wenigen Minuten und ca. 45 Minuten auf dem Haar zu belassen. Anschließend wird das Haar dann mit Wasser oder einem tensidhaltigen Mittel ausgespült.

Der zweite Schritt des erfindungsgemäßen Verfahrens, in dem das den UV-Filter enthaltende Mittel aufgetragen wird, kann sein:

- Das oben genannte Ausspülen des Färbemittels, wobei das Wasser oder die tensidische Zubereitung zusätzlich einen UV-Filter enthält.
- Das Aufbringen eines weiteren Mittels mit einem UV-Filter im Anschluß an den genannten Spülvorgang, wobei dieses weitere Mittel nach einer Einwirkzeit von wenigen Sekunden bis etwa 20 Minuten ebenfalls wieder aus dem Haar entfernt wird. In diesem Fall kann es sich beispielsweise um ein Haarbehandlungsmittel vom Typ einer Spülung oder eines Conditioners handeln.
- Das Aufbringen eines weiteren Mittels mit einem UV-Filter im Anschluß an den genannten Spülvorgang, wobei dieses weitere Mittel auf dem Haar verbleibt. In diesem Fall kann es sich beispielsweise um ein Haarbehandlungsmittel vom Typ eines Conditioners, einer Haarkur oder ein Haarspray handeln.

Dieses zweite Mittel, das dem Aufbringen des UV-Filters auf das Haar dient, kann jeweils die für den Typ des gewählten Mittel üblichen weiteren Bestandteile enthalten. Es wird in diesem Zusammenhang sowohl ausdrücklich auf das Grundwissen des einschlägigen Fachmanns, dokumentiert beispielsweise durch die oben genannte Monographie von Schrader, als auch auf die oben im Rahmen der Beschreibung der Färbemittel aufgeführten weiteren Bestandteile verwiesen. Auch die Art der Konfektionierung dieses zweiten Mittels unterliegt prinzipiell keinen Einschränkung. Cremes, Lotionen, Lösungen, Emulsionen, Gele, Sprays und tensidhaltige schäumende Lösungen, z. B. Shampoos oder Schaumaerosole sind geeignet. Wegen der damit häufig verbundenen Einschränkungen hinsichtlich der weiteren Bestandteile kann die Formulierung in Form einer sogenannten PIT-Emulsion allerdings weniger bevorzugt sein.

Schließlich wurde eine besonders hohe Verbesserung der Waschechtheit von Färbungen auf keratinischen Fasern gefunden, wenn ein Mittel eingesetzt wurde, das eine Kombination aus einem wasserunlöslichen UV-Filter, einem UV-Filter gemäß Formel (I) sowie einem Mono-, Di- oder Oligosaccharid enthielt.

Ein weiterer Gegenstand der Erfindung ist somit ein Mittel zur Erhöhung der Waschechtheit von Färbungen auf keratinischen Fasern, dadurch gekennzeichnet, daß es eine Wirkstoffkombination enthält, die aus mindestens einem wasserunlöslichen UV-Filter. mindestens einem UV-Filter der Formel (I) sowie einem Mono-, Di- oder Oligosaccharid besteht.

Gemäß dem oben gesagten kann es sich bei diesem Mittel sowohl um das eigentliche Färbemittel handeln als auch um ein Mittel, das nach dem eigentlichen Färbevorgang in einem zweiten Schritt auf das Haar aufgebracht wird. Bezüglich der bevorzugten Ausführungsformen hinsichtlich der zwingenden Bestandteile sowie der weiteren fakultativen Komponenten dieser erfindungsgemäßen Mittel wird ausführlich auf das oben gesagte verwiesen.

Die folgenden Beispiele sollen den Erfindungsgegenstand weiter erläutern.

**Beispiele**

Alle Mengenangaben in den folgenden Beispielen sind, sofern nicht anders vermerkt, Gewichtsteile.

| Rezeptur Färbemittel | |
|---|---|
| Ölsäure | 6,5 |
| Propylenglykol | 6,5 |
| Isopropanol | 12,0 |
| Natriumlaurylethersulfat + 2EO (27 % Aktivsubstanz in Wasser) | 3,5 |
| Natriumsulfit | 0,2 |
| EDTA-Dinatriumsalz | 0,1 |
| p-Aminophenol HCl | 0,5 |
| p-Toluylendiamin-sulfat | 0,1 |
| Resorcin | 0,2 |
| 4-Amino-2-nitro-diphenylamin-2'-carbonsäure | 0,1 |
| 6-Nitro-1,2,3,4-tetrahydrochinoxalin | 0,1 |
| 5-Amino-2-methylphenol | 0,3 |
| Parfüm | 0,5 |
| Monoethanolamin ad | pH 9,2 |
| Wasser ad | 100 |

Das Färbemittel wurde mit einem handelsüblichen Entwickler (Poly Country Colors) mit 5 % Wasserstoffperoxid im Verhältnis Färbemittel:Entwickler von 40ml:50ml gemischt.

| Rezepturen Conditioner | | | |
|---|---|---|---|
| | B1 | B2 | V1 |
| Fettalkohol-C16/18 (1:1) | 3,0 | 3,0 | 3,0 |
| Paraffinöl perliq. | 3,0 | 3,0 | 3,0 |
| Cetyltrimethylammoniumchlorid | 0,8 | 0,8 | 0,8 |
| Parfüm | 0,3 | 0,3 | 0,3 |
| Escalol® HP610[1] | 1,0 | - | - |
| Uvinul® M 40[2] | - | 0,5 | - |
| Wasser | <---------------ad100----------------> | | |

[1] Dodecyl-dimethylaminobenzamidopropyl-dimethylammoniumtosylat (ISP)
[2] 2-Hydroxy-4-methoxy-benzophenon (INCI-Bezeichnung: Benzophenone-3) (BASF)

Die Ausfärbungen wurden auf naturweißen Haarsträhnen (ca. 2 g) des Typs Alkinco Virgin White durchgeführt. Dazu wurden 8 g der Färbemittel/Entwicklermischung auf die Haarsträhne aufgebracht, 30 Minuten auf der Strähne belassen und dann mit Wasser (37° C) ausgespült. Danach wurde die Strähne 2 Minuten lang mit dem Conditioner (B 1, B2 bzw. V1) behandelt, anschließend 6 mal mit je 0,5 ml eines handelsüblichen Shampoos gewaschen, mit Wasser (37° C) gespült und getrocknet.

Anschließend wurde die Farbintensität der Haarsträhne von Fachleuten im Rahmen des folgenden Schemas bewertet:

Farbintensität = 1: Strähne Virgin White vor der Färbung
Farbintensität = 6: Strähne nach dem Färben Spülen mit Wasser (37°C) und Trocknen. (Keine Behandlung mit dem Conditioner).

Es ergaben sich folgende Bewertungen:

| Conditioner | Bewertung |
|---|---|
| B1 | 5 |

(fortgesetzt)

| Conditioner | Bewertung |
|---|---|
| B2 | 5 |
| V1 | 4 |

Die Waschechtheit der Färbungen war somit auf den erfindungsgemäß nachbehandelten Strähnen signifikant höher als auf den mit dem Vergleichsmittel nachbehandelten Strähnen.

## Patentansprüche

1. Verwendung eines UV-Filters zur Verbesserung der Waschechtheit von Färbungen keratinischer Fasern.

2. Verwendung nach Anspruch 1, **dadurch gekennzeichnet, daß** es sich um einen wasserunlöslichen UV-Filter handelt.

3. Verwendung nach Anspruch 1 oder 2, **dadurch gekennzeichnet, daß** der UV-Filter eine Benzophenon-Einheit aufweist.

4. Verwendung nach Anspruch 1, **dadurch gekennzeichnet, daß** der UV-Filter eine kationische Gruppe aufweist.

5. Verwendung nach Anspruch 4, **dadurch gekennzeichnet, daß** der UV-Filter eine allgemeine Struktur gemäß Formel (I) aufweist,

$$U - Q \qquad (I)$$

in der U für eine UV-Strahlen absorbierende Gruppe steht und Q für eine Gruppe, die mindestens eine quartäre Ammoniumfunktion enthält.

6. Verwendung nach Anspruch 5, **dadurch gekennzeichnet, daß** die Gruppe Q gemäß Formel (I) die allgemeine Struktur $-(CH_2)_X-N^+R^1R^2R^3\ X^-$ aufweist, in der x steht für eine ganze Zahl von 1 bis 4, $R^1$ und $R^2$ unabhängig voneinander stehen für $C_{1-4}$-Alkylgruppen, $R^3$ steht für eine $C_{1-22}$-Alkylgruppe oder eine Benzylgruppe und $X^-$ für eine physiologisch verträgliche anionische Gruppe.

7. Verwendung nach Anspruch 6, **dadurch gekennzeichnet, daß** mindestens zwei der Gruppen $R^1$, $R^2$ und $R^3$ Methylgruppen sind.

8. Verwendung nach einem der Ansprüche 5 bis 7, **dadurch gekennzeichnet. daß** die Gruppe U so ausgewählt ist, daß der UV-Filter ein Absorptionsmaximum im UVB-Bereich aufweist.

9. Verwendung nach einem der Ansprüche 1 bis 8, **dadurch gekennzeichnet, daß** der UV-Filter einen molaren Extinktionskoeffizienten am Absorptionsmaximum von mindestens 15000 aufweist.

10. Verwendung nach einem der Ansprüche 1 bis 9, **dadurch gekennzeichnet, daß** der UV-Filter in Kombination mit einem Mono-, Di- oder Oligosaccharid eingesetzt wird.

11. Verwendung nach einem der Ansprüche 1 bis 10, **dadurch gekennzeichnet, daß** der UV-Filter in Kombination mit einem Spreitmittel eingesetzt wird.

12. Verwendung nach Anspruch 11, **dadurch gekennzeichnet, daß** das Spreitmittel Paraffinöl ist.

13. Verfahren zur Färbung keratinischer Fasern, **dadurch gekennzeichnet, daß** in einem ersten Schritt die keratinische Faser in üblicher Weise gefärbt wird und in einem zweiten Schritt ein Mittel aufgetragen wird, das mindestens einen UV-Filter zur Erhöhung der Waschechtheit der Färbung enthält und gewünschtenfalls nach einer Einwirkzeit von wenigen Sekunden bis etwa 20 Minuten wieder ausgespült wird.

**14.** Verfahren zur Färbung keratinischer Fasern in üblicher Weise mit einem Färbemittel, **dadurch gekennzeichnet, daß** das Färbemittel zur Erhöhung der Waschechtheit der Färbung einen UV-Filter enthält.

**15.** Mittel zur Erhöhung der Waschechtheit von Färbungen keratinischer Fasern, **dadurch gekennzeichnet, daß** es eine Wirkstoffkombination enthält, die besteht aus

- mindestens einem wasserunlöslichen UV-Filter,
- mindestens einem UV-Filter der Formel (I) gemäß Anspruch 5 sowie
- einem Mono-, Di- oder Oligosaccharid.

**Claims**

**1.** The use of a UV filter for improving the fastness to washing of colours on keratin fibres.

**2.** The use claimed in claim 1, **characterized in that** the UV filter is insoluble in water.

**3.** The use claimed in claim 1 or 2, **characterized in that** the UV filter contains a benzophenone unit.

**4.** The use claimed in claim 1, **characterized in that** the UV filter contains a cationic group.

**5.** The use claimed in claim 4, **characterized in that** the UV filter has a general structure corresponding to formula (I):

$$U - Q \qquad (I)$$

in which U is a UV-absorbing group and Q is a group containing at least one quaternary ammonium function.

**6.** The use claimed in claim 5, **characterized in that** the group Q in general formula (I) has the general structure $-(CH_2)_xN^+R^1R^2R^3\ X^-$, where x is an integer of 1 to 4, $R^1$ and $R^2$ independently of one another represent $C_{1-4}$ alkyl groups, $R^3$ is a $C_{1-22}$ alkyl group or a benzyl group and $X^-$ is a physiologically compatible anionic group.

**7.** The use claimed in claim 6, **characterized in that** at least two of the groups $R^1$, $R^2$ and $R^3$ are methyl groups.

**8.** The use claimed in any of claims 5 to 7, **characterized in that** the group U is selected so that the UV filter has an absorption maximum in the UVB range.

**9.** The use claimed in any of claims 1 to 8, **characterized in that** the UV filter has a molar extinction coefficient at the absorption maximum of at least 15,000.

**10.** The use claimed in at least one of claims 1 to 9, **characterized in that** the UV filter is used in combination with a mono-, di- or oligosaccharide.

**11.** The use claimed in any of claims 1 to 10, **characterized in that** the UV filter is used in combination with a spreading agent.

**12.** The use claimed in claim 11, **characterized in that** the spreading agent is paraffin oil.

**13.** A process for colouring keratin fibres, **characterized in that**, in a first step, the keratin fibre is coloured in the usual way and, in a second step, a preparation containing at least one UV filter to increase the fastness of the colour to washing is applied and, if desired, is rinsed out again after a contact time of a few seconds to about 20 minutes.

**14.** A process for colouring keratin fibres in the usual way with a colorant, **characterized in that** the colorant contains a UV filter to increase the fastness of the colour to washing.

**15.** A composition for increasing the fastness to washing of colours on keratin fibres, **characterized in that** it contains a combination of active substances consisting of

- at least one water-insoluble UV filter
- at least one UV filter corresponding to formula (I) in claim 5 and
- a mono-, di- or oligosaccharide.

**Revendications**

1. Utilisation d'un filtre UV pour améliorer la résistance au lavage des colorations de fibres kératiniques.

2. Utilisation selon la revendication 1, **caractérisée en ce qu'**il s'agit d'un filtre UV insoluble dans l'eau.

3. Utilisation selon la revendication 1 ou 2, **caractérisée en ce que** le filtre UV présente une unité benzophénone.

4. Utilisation selon la revendication 1, **caractérisée en ce que** le filtre UV présente un groupe cationique.

5. Utilisation selon la revendication 4, **caractérisée en ce que** le filtre UV présente une structure générale selon la formule (I),

$$U - Q \qquad (I)$$

dans laquelle U représente un groupe absorbant les rayons UV et Q représente un groupe qui contient au moins une fonction ammonium quaternaire.

6. Utilisation selon la revendication 5, **caractérisée en ce que** le groupe Q selon la formule (I) présente la structure générale $-(CH_2)_x-N^+R^1R^2R^3X^-$ , dans laquelle x représente un nombre entier allant de 1 à 4, $R^1$ et $R^2$ représentent indépendamment l'un de l'autre des groupes alkyle en $C_1$ à $C_4$, $R^3$ représente un groupe alkyle en $C_1$ à $C_{22}$ ou un groupe benzyle et $X^-$ représente un groupe anionique physiologiquement acceptable.

7. Utilisation selon la revendication 6, **caractérisée en ce qu'**au moins deux des groupes $R^1$, $R^2$ et $R^3$ sont des groupes méthyle.

8. Utilisation selon l'une des revendications 5 à 7, **caractérisée en ce que** le groupe U est choisi de manière que le filtre UV présente un maximum d'absorption dans le domaine UVB.

9. Utilisation selon l'une des revendications 1 à 8, **caractérisée en ce que** le filtre UV présente un coefficient d'extinction molaire au maximum d'absorption d'au moins 15 000.

10. Utilisation selon l'une des revendications 1 à 9, **caractérisée en ce que** le filtre UV est utilisé en combinaison avec un mono-, un di- ou un oligosaccharide.

11. Utilisation selon l'une des revendications 1 à 10, **caractérisée en ce que** le filtre UV est utilisé en combinaison avec un agent de déploiement.

12. Utilisation selon la revendication 11, **caractérisée en ce que** l'agent de déploiement est une huile de paraffine.

13. Procédé de coloration de fibres kératiniques, **caractérisé en ce que** dans une première étape on colore les fibres kératiniques de manière habituelle et **en ce que**, dans une seconde étape, on introduit un agent qui contient au moins un filtre UV pour augmenter la résistance au lavage de la coloration et le cas échéant **en ce qu'**on le rince à nouveau au bout d'un temps d'action de quelques secondes à environ 20 minutes.

14. Procédé de coloration de fibres kératiniques de manière habituelle avec un colorant, **caractérisé en ce que** le colorant contient un filtre UV pour augmenter la résistance au lavage de la coloration.

15. Agent pour augmenter la résistance au lavage de colorations de fibres kératiniques, **caractérisé en ce qu'**il contient une combinaison de matières actives qui se compose de

    - au moins un filtre UV insoluble dans l'eau,

- au moins un filtre UV de formule (1) selon la revendication 5 ainsi que
- un mono-, di- ou oligosaccharide.